# EUROPEAN PATENT SPECIFICATION

(11) **EP 4 308 547 B1**
(45) Date of publication and mention of the grant of the patent: **19.02.2025**
(21) Application number: 22712368.4
(22) Date of filing: 09.03.2022
(51) Int. Cl.: C07D 209/12, G01N 33/68

(54) **HIGHLY WATER-SOLUBLE AND STABLE CHEMOSENSOR FOR CYSTEINE**
HOCH WASSERLÖSLICHER UND STABILER CHEMOSENSOR FÜR CYSTEIN
CAPTEUR CHIMIQUE HAUTEMENT HYDROSOLUBLE ET STABLE POUR LA CYSTÉINE

(30) Priority: 15.03.2021 EP 21162688
(43) Date of publication of application: 24.01.2024
(73) Proprietor: F. Hoffmann-La Roche AG, 4070 Basel (CH); Roche Diagnostics GmbH, 68305 Mannheim (DE)
(72) Inventor: FOMIN, Maksim, 82377 Penzberg (DE); KUCHELMEISTER, Hannes, 82377 Penzberg (DE)
(74) Representative: Krauss, Jan
(86) International application number: PCT/EP2022/056065
(87) International publication number: WO 2022/194645

(56) References cited:
- EP-A2- 3 779 449
- WO-A1-2015/198341
- WO-A1-2016/166773

## Description

The present invention relates to chemical probes for the improved detection of cysteine in a test sample, preferably an aqueous test sample, as well as respective uses and kits.

### Background of the invention

Cysteine (Cys) is important in biosynthesis, detoxification, and metabolism. An elevated level of total cysteine can predict cardiovascular disease and metabolic syndromes. Cysteine deficiency is known to be one of the consequences of aging. The selective detection of Cys over structurally similar homocysteine (Hcy) or glutathione (GSH) remains an immense challenge. Although there are many methods for detecting Cys, photoluminescence (PL) and electrochemiluminescence (ECL) techniques are well-suited for clinical diagnostics and analytical technology because of their high sensitivities.

Trisulfide formation in recombinant monoclonal antibodies is a source of heterogeneity which needs to be controlled for consistent product quality. Ryll *et al.* (Kshirsagar, R.; McElearney, K.; Gilbert, A.; Sinacore, M.; Ryll, T. Biotechnol. Bioeng. 2012, 109, 2523) have shown that the L-cysteine (Cys) concentration in the feed medium directly correlated with the trisulfide level in the product (IgG1 mAb). Therefore, controlling of Cys feed strategies is required to lower trisulfide formation to acceptable levels.

Up to now, the detection of Cys attracts a lot of attention for various biochemical applications. A number of methods for detecting Cys have been developed such as fluorometry, potentiometry, electrochemical voltammetry and HPLC combined with Ellman's reagent or coupled with fluorescence. These methods require complicated instrumentation, involve cumbersome laboratory procedures or are low throughput.

Kim and Hong (in: Photoluminescence and Electrochemiluminescence Dual-Signaling Sensors for Selective Detection of Cysteine Based on Iridium(III) Complexes. ACS Omega 2019, 4, 7, 12616-12625) report PL and ECL dual-channel sensors using cyclometalated iridium(III) complexes for the discrimination of Cys from Hcy and GSH.

UV-vis spectrometry provides fast and simple measurement procedures. Thus, to quantify essential metabolites in bioprocesses, photometric assays are employed using automated analyzers, such as Cedex Bio HT (Roche Diagnostics, Penzberg, Germany). But only few candidates can be utilized for colorimetric Cys detection on Cedex Bio HT Analyzer ("Cedex"), and the analytical device offers only a limited set of wavelengths: 340, 378, 409, 480, 512, 520, 552, 583, 629, 652, 659, and 800 nm. In addition, an ideal probe for Cedex system must comprise high sensitivity, rapid response, aqueous solubility and stability, and ease of use.

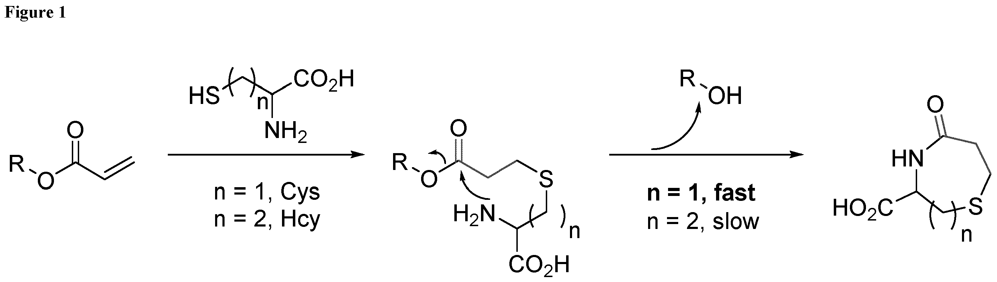

To date, most of the indicators for Cys are based on the strong nucleophilicity of the thiol group. Various mechanisms have been employed, including Michael addition and cleavage reactions. A sensing strategy based on acrylate group seemed promising, because it allowed to discriminate Cys from other amino acids and thiols (Han, Q.; Shi, Z.; Tang, X.; Yang, L.; Mou, Z.; Li, J.; Shi, J.; Chen, C.; Liu, W.; Yang, H.; Liu, W. Organic & Biomolecular Chemistry 2014, 12, 5023). The sensing mechanism is given in Figure 1. This strategy involves the conjugate addition of Cys to acrylate to generate thioesters followed by an intramolecular cyclization. The acrylate moiety as thiol activation site undergoes fast cyclization only with Cys, since the reaction rate depends strongly on the ring size of the resulting lactam. After the masking acrylate group is removed, the conjugated π-electron system of the chromophore is restored, which enables the colorimetric response.

Disadvantageously, only few acrylate-based probes feature an intense colorimetric response at the wavelength required for the use on Cedex. Their chromophores are based on xanthene, merocyanine, heptamethine and fluorescein. The fact that most of reported acrylates were applied in organic solvent-water mixtures is obviously necessitated by their poor solubility in aqueous media. However, it is a must for applications on Cedex that probes are soluble in water, since parts of the instrument are labile to organic solvents. Also, the stability of existing probes has to be evaluated in order to ensure that an assay solution can be stored in Cedex for reasonable amount of time.

In view of these and other disadvantages, it is an object of the present invention to provide Cys probes that which can be used in aqueous solutions and provide a sufficient colorimetric response at one of the required wavelengths. Other objects and advantages will become apparent to the person of skill when studying the present description of the present invention.

In a first aspect of the present invention, the above object is solved by a compound according to Formula (I), 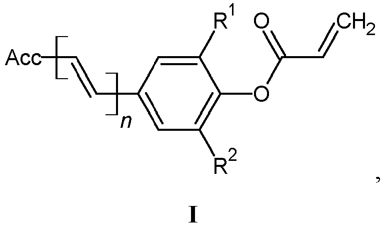 wherein
R¹ and R² are independently selected from R³, O-R³, S-R³, SO₃⁻, SO₃-R³, wherein R³ is selected from Ci-Cis alkyl, and a polyethylene glycol (PEG) residue,
Acc is selected from the group selected from Formula II 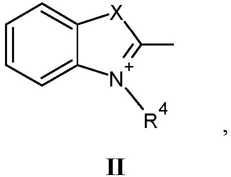
wherein X is selected from -N(CH₃)-, -S-, -Se-, -O-, and -C(CH₃)₂-, 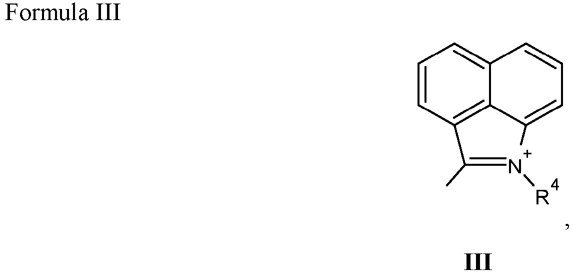 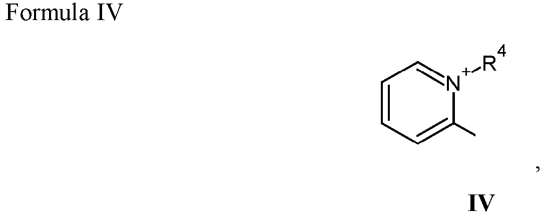 and 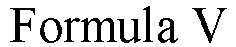 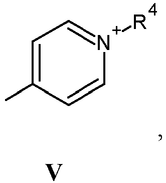
wherein optionally in each of Formula II to V an aromatic ring is substituted with 1, 2 or 3 SO₃⁻ groups,
R⁴ is selected from the group of C₁-C₁₈ alkyl, C₁-C₆ cycloalkyl, and (CH₂)ₘ-SO₃⁻, wherein m is an integer selected from 1 to 18, and n is selected from 1, 2 and 3, and suitable salts and solvates thereof.

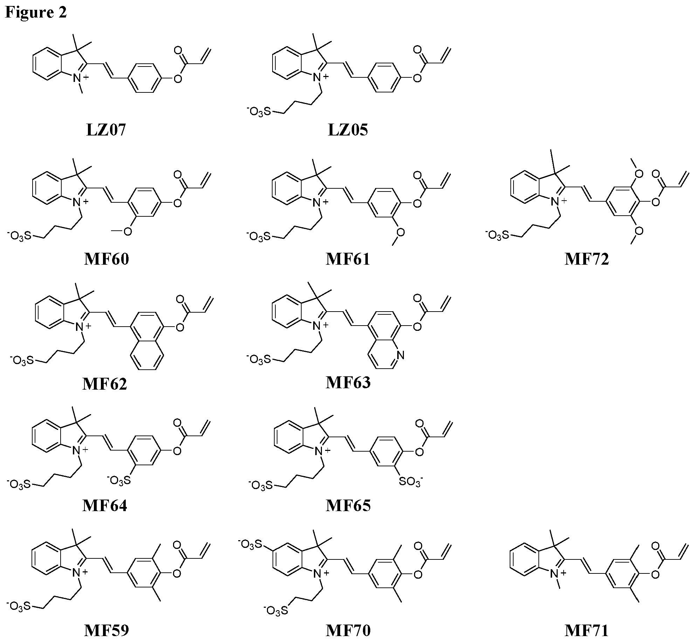

The present inventors synthesized a series of acryloyl esters based on merocyanine chromophore (Figure 2, see below); probe **LZ07** was prepared as a control and for comparison, and is known from the literature (Han, Q.; Shi, Z.; Tang, X.; Yang, L.; Mou, Z.; Li, J.; Shi, J.; Chen, C.; Liu, W.; Yang, H.; Liu, W. Organic & Biomolecular Chemistry 2014, 12, 5023). The present inventors then studied the spectral properties, aqueous solubility and stability of the acryloyl esters, and evaluated the response to Cys. It could be demonstrated that the chemical design provided dedicated probes, in particular for Cedex.

Preferred is the compound of Formula I according to the present invention, wherein R¹ and R² are independently selected from R³, O-R³, S-R³, SO₃⁻, SO₃-R³, wherein R³ is selected from C₁-C₆ alkyl, and a polyethylene glycol (PEG) residue,
Acc is Formula II 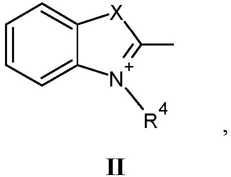 wherein X is selected from -N(CH₃)-, -S-, -Se-, -O-, and -C(CH₃)₂-, optionally the aromatic ring is substituted with 1, 2 or 3 SO₃⁻ groups, R⁴ is selected from C₁-C₆ alkyl, C₁-C₆ cycloalkyl, and (CH₂)ₘ-SO₃⁻, wherein m is an integer from 1 to 6, and n is 1, and suitable salts and solvates thereof.

Further preferred is the compound of Formula I according to the present invention, wherein R¹ and R² are independently selected from R³, O-R³, S-R³, SO₃⁻, SO₃-R³, wherein R³ is selected from C₁-C₃ alkyl, and a polyethylene glycol (PEG) residue,
Acc is Formula II 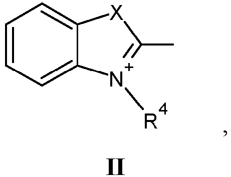 wherein X is -C(CH₃)₂-, optionally the aromatic ring is substituted with 1, 2 or 3 SO₃⁻ groups, R⁴ is (CH₂)ₘ-SO₃⁻, wherein m is an integer from 1 to 6, and n is 1, and suitable salts and solvates thereof.

Further preferred is the compound of Formula I according to the present invention according to the following formulae VI to IX 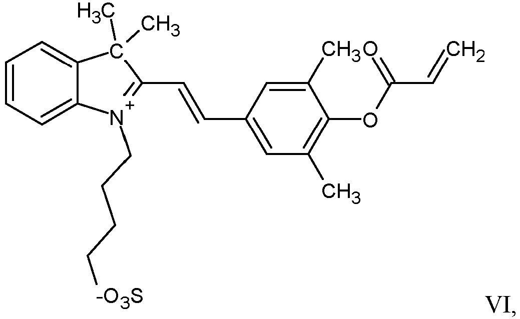 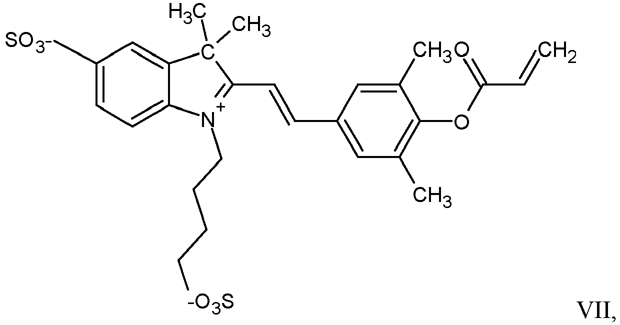 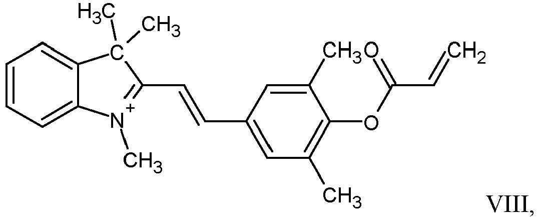 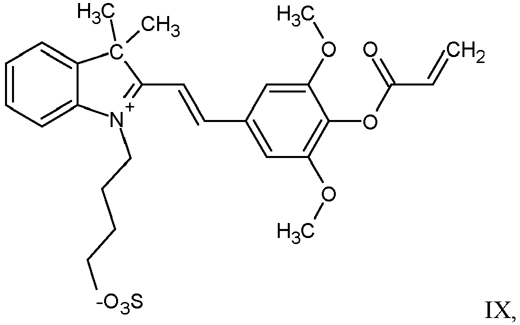 and suitable salts and solvates thereof.

In the context of the present invention, a suitable salt is usually one that does not interfere or does not substantially interfere with the solubility of the compound according to the present invention, in particular with the solubility in aqueous media. Examples are salts containing Group I elements (Li⁺, Na⁺, K⁺, Cs⁺, Rb⁺), the ammonium ion (NH₄⁺), the nitrate ion (NO₃⁻), containing Cl ⁻, Br ⁻, or I ⁻, or sulfate salts.

Yet another aspect of the present invention then relates to a method for preparing a compound according to Formula I according to the present invention, comprising the steps of:
a) suitably reacting a compound of Formula VI 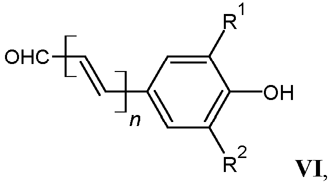 wherein R¹ and R² are as defined as above, and n is 1 or 2, with a compound of Formula II 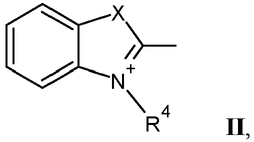 or with a compound of formula III 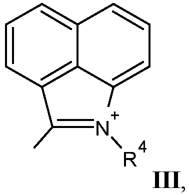 or with a compound of Formula IV 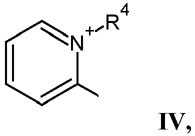 or with a compound of Formula V 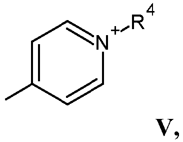 wherein in each of Formulae II to V optionally an aromatic ring is substituted with 1, 2 or 3 SO₃⁻ groups, R⁴ is selected from C₁-C₁₈ alkyl, C₁-C₆ cycloalkyl, and (CH₂)ₘ-SO₃⁻, and wherein m is an integer from 1 to 18, to obtain a compound of Formula VIII 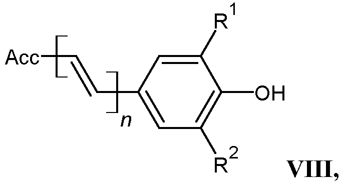 wherein R¹, R² and Acc are as defined as above, and n is 1 or 2, and b) suitably reacting the compound of formula VIII with acryloyl chloride. Suitable conditions for performing the above method are known to the person of skill in the art, and are exemplary disclosed in the examples and schemes, below.

Yet another aspect of the present invention then relates to a method for detecting cysteine in a test sample, comprising the following steps of a) Measuring of UV/Vis absorbance of a solution of a compound as defined according to the present invention in a suitable solvent before and after being contacted with a prospectively cysteine-containing test sample, and b) Determining the difference in absorbance by comparison of the UV/Vis spectra as measured in step a), and c) Detecting cysteine in said test sample based on said difference in absorbance as determined in step b).

Test samples according to the present invention can comprise any sample comprising or prospectively comprising cysteine. Examples are, for example, the detection of biothiols in plasma, in samples obtained from patients, in total proteins in different kinds of cell lines, in tissue samples, cell lysates, serum, saliva or urine, in antibody samples, and in samples used in biotechnological applications. Preferred are aqueous biological samples to be analyzed in a Cedex-system.

Spectra recorded in the presence of Cys confirmed a colorimetric response through the cleavage of acryloyl ester. Probes as synthesized showed significant bathochromic shifts to the green and yellow range of the visible spectrum (Table 2). Preferred is the method according to the present invention, wherein the UV/Vis absorbance is measured at discrete wavelengths in the range of from 200 nm to 1000 nm. Moreover, their spectral profiles were advantageously fulfilling the wavelength requirement for the Cys sensing application in Cedex system (340, 378, 409, 480, 512, 520, 552, 583, 629, 652, 659 and 800 nm).

More preferred is the method according to the present invention, wherein the solvent is an aqueous solvent.

In a preferred aspect of the present invention, the method according to the present invention, wherein said determining the difference in absorbance is by a visual inspection of a color change, such a significant bathochromic shift to the green and yellow range of the visible spectrum. As an example, in a slightly different approach, Hai-Feng Yin, et al. (in: simple probe with visible color change for selective detection of cysteine, Spectroscopy Letters, (2020) DOI: 10.1080/00387010.2020.1821063) synthesized a fluorescent probe, which can selectively detect cysteine. With addition of cysteine, the probe solution showed marked color change from pale yellow to orange color by the naked eye.

The method according to the present invention, wherein said determining the difference in absorbance is by Cedex Bio HT (Roche Diagnostics, Penzberg, Germany).

Yet another aspect of the present invention then relates to a kit for detecting cysteine in a test sample, comprising a vial or container comprising a predetermined quantity of a compound according to the present invention, together with a manual for using said kit. Examples of included materials are, for example, a standard, a probe according to the invention, and buffer(s).

Yet another aspect of the present invention then relates to the use of a compound according to the present invention, or a kit according to the present invention for detecting cysteine in a test sample, preferably an aqueous test sample as disclosed herein.

The present invention will now be described further in the following examples, and also with reference to the Figures, nevertheless, without being limited thereto. For the purposes of the present invention, all references as cited herein are incorporated by reference in their entireties.
Figure 1 shows a scheme of the mechanism of reaction of acryloyl esters with Cys (R-OH = merocyanine).
Figure 2 shows the structure of probes as synthesized in the context of the present invention.
Figure 3 shows the results of calibration in a feed medium (DMT118F.01 w/o Cys). SR: **MF70** in DMSO/water (1:1). R1: 100mM K-PO₄.

### EXAMPLES

A series of acryloyl esters based on merocyanine chromophore was synthesized (Figure 2), and was compared with probe LZ07 as known from the literature (Han, Q.; Shi, Z.; Tang, X.; Yang, L.; Mou, Z.; Li, J.; Shi, J.; Chen, C.; Liu, W.; Yang, H.; Liu, W. Organic & Biomolecular Chemistry 2014, 12, 5023). Spectral properties, aqueous solubility and stability were studied, and the response to Cys was evaluated. The inventors demonstrated that the chemical design according to the present invention provides dedicated probes for Cedex.

The following is a brief summary of the state of the art regarding know Cys-probes and their properties:

| **Probe** | **Solvent** | **Spectra** | **Reference** |
|---|---|---|---|
| 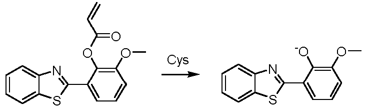 | Assay EtOH/H2O (2:8, v/v) solution buffered at pH 7.4 (phosphate buffer, 20 mm) | | Yang, X.; Guo, Y; Strongin, R. M. Angew. Chem. Int. Ed. 2011, 50, 10690. |
| 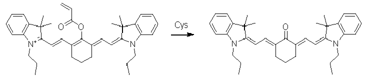 | Assay EtOH : HEPES (1:9, pH 7.4, 0.01 M) | Probe 775 nm | Guo, Z.; Nam, S.; Park, S.; Yoon, J. Chemical Science 2012, 3, 2760. |
| | | With Cys 515 nm | |
| 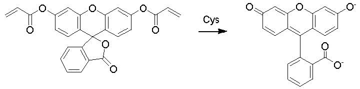 | Assay ethanol-phosphate buffer (20 mM, pH 7.4, 2 : 8 v/v) | With Cys 490 nm | Wang, H.; Zhou, G.; Gai, H.; Chen, X. Chem. Commun. 2012, 48, 8341. |
| 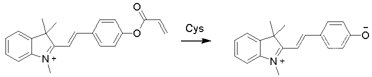 | | Probe 384 nm | Han, Q.; et al. Organic & Biomolecular Chemistry 2014, 12, 5023. |
| | | With Cys 512 nm | |
| 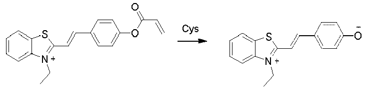 | | | Li, H.; Jin, L.; Kan, Y; Yin, B. Sensors and Actuators B: Chemical 2014, 196, 546. |
| 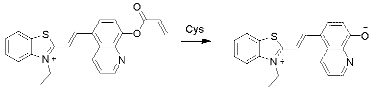 | | Probe 387 nm | |
| | | With Cys 566 nm | |
| 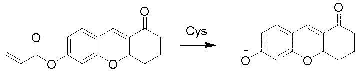 | | Probe 302, 358 nm | Lee, Y. H.; et al. Chem. Commun. 2015, 51, 14401. |
| | | With Cys 398 nm | |
| 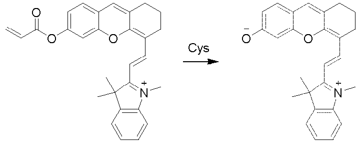 | | Probe 582 nm | Zhang, J.; et al. Anal. Chem. 2015, 87, 4856. |
| | | With Cys 674 nm | |
| 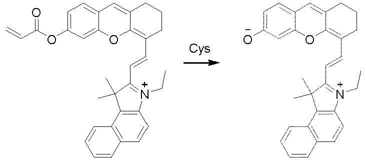 | | Probe 608, 572 nm | Han, C.; et al. ACS Applied Materials & Interfaces 2015, 7, 27968. |
| | | With Cys 710, 684, 632 nm | |
| | | | |
| 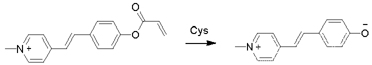 | | Probe 335 nm | Niu, W; et al. Anal. Chem. 2016, 88, 1908. |
| | | With Cys 372 nm | |
| 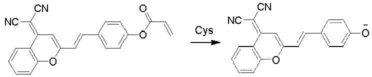 | | Probe 381 nm | Wang, J.; et al. ACS Sensors 2016**.** |
| | | With Cys 557 nm | |
| 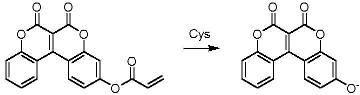 | Stock solution DMSO | Probe 350 nm | Chen, C.; Zhou, L.; Huang, X.; Liu, W. Journal of Materials Chemistry B 2017, 5, 5892. |
| | | With Cys 445 nm | |
| | Assay DMSO-PBS | | |
| 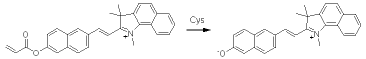 | PBS | With Cys 460 nm | Dai, X.; Kong, X.; Lin, W. Dyes and Pigments 2017, 142, 306. |
| 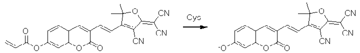 | Stock solution DMSO | Probe 423 nm | Feng, S.; et al. Dyes and Pigments 2017, 146, 103. |
| | | With Cys 335, 584 nm | |
| | Assay PBS buffer solution (10 mM, pH 7.4, with 10% DMSO, v/v) | | |
| 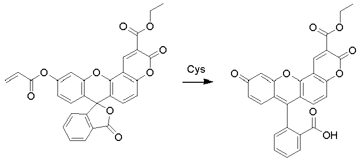 | Assay DMSO-PBS buffer solution (10.0 mM, pH = 7.4, 3:7 (v/v)) | Probe 332 nm | Fu, Z.-H.; et al. Anal. Chem. 2017, 89, 1937. |
| | | With Cys 450 nm, | |
| 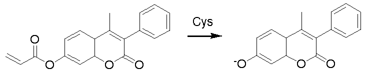 | Assay PBS containing 10% CH3CN | Probe 315 nm | Kang, Y-F.; et al. Aust. J. Chem. 2017, 70, 952. |
| | | With Cys 380 nm | |
| 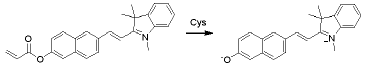 | Assay PBS containing 1% DMSO | Probe 409 nm | Liu, G.; et al. J. Talanta 2017, 170, 406. |
| | | With Cys 448 nm | |
| 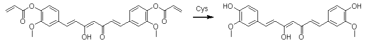 | Assay phosphate buffer-C2H5O H (pH 7.4, v/v, 1: 1) | Probe 400 nm | Pang, L.; et al. Industrial & Engineering Chemistry Research 2017, 56, 7650. |
| | | With Cys 427 nm. | |
| 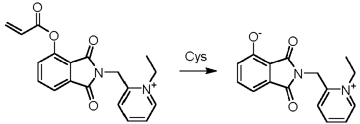 | Assay 50 mM PBS solution (THF/water = 1:9, pH 7.4) | Probe 360 nm With Cys 400 nm | Shen, Y; et al. Spectrochim. Acta Mol. Biomol. Spectrosc. 2017, 185, 371. |
| 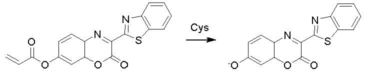 | Assay DMSO :H2O = 4 : 1 v/v, 10 mM HEPES buffer, pH = 7.4 | Probe 386 nm | Manna, S.; et al. New J. Chem. 2018, 42, 4951. |
| | | With Cys 527 nm | |
| 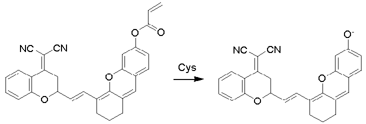 | PBS buffer (10 mM, pH = 7.4, with 50% of DMSO, v/v) | Probe 550 nm | Qi, Y; et al. Anal. Chem. 2018, 90, 1014. |
| | | With Cys 600 nm | |

### Experimental Procedures

### Materials and Methods

Reagents and solvents were purchased at the highest commercial quality from Sigma-Aldrich and used without further purification. CHROMASOLV solvents were used as eluents in HPLC. Yields refer to chromatographically (HPLC-MS) and spectroscopically (¹H NMR) homogeneous material, unless otherwise stated. Counter anions are omitted for clarity.

### Analytical HPLC-MS (ESI-MS)

The purity of the compounds was determined with the help of an HPLC-MS apparatus from Waters (Milford, USA) containing the following components: 2695 Separation module, 2696 photodiode array and Waters Micromass ZQ (ESCI ionization mode) detectors. Data acquisition was carried out by MassLynx (V4.1) software.

Column: YMC-Triart C18 3 µM (4.6 x 150 mm)/Product Nr.TA12S03-1546WT.

Flow: 0.7 mL/min.

Phase A: triethylammonium acetate (TEAAc) buffer (10 mM, pH 7.0) in deionized water.

Phase B: MeCN.

Gradient 80: 5-80B (7 min); 80-80B (2 min); 80-5B (0.5 min); 5-5B (2.5 min).

Gradient 100: 5-100B (7 min); 100-100B (2 min); 100-5B (0.5 min); 5-5B (2.5 min).

### NMR

NMR spectra were recorded on a Bruker Avance (500 and 600 MHz) and an Agilent 400 MR DD2 (400 MHz) instruments and were calibrated using residual non-deuterated solvent as an internal reference.¹ The following abbreviations were used to explain NMR peak multiplicities: s = singlet, d = doublet, t = triplet, q = quartet, m = multiplet, br = broad.

### HRMS

For HRMS (high resolution mass spectra), samples were dissolved in MeCN and analyzed by direct-flow injection (injection volume = 5 µL) electrospray ionization time-of-flight (ESI-TOF) mass spectrometry on a Waters Q-ToF Premier instrument in the positive ion mode.

### General procedure I

### Preparation of merocyanine dyes

A mixture of a respective aldehyde (1 equiv.) and an indolium salt (1 equiv.) in ethanol was refluxed for 1-16 h in the presence of piperidine (0.1-2 equiv.) under Ar. The reaction mixture was allowed to cool slowly to rt, solvent was removed *in vacuo* and the residue was purified by reversed phase column chromatography (C-18, TEAB buffer (10 mM, pH 7.4)/MeCN or H₂O (0.1% TFA)/MeCN).

### General procedure II

### Preparation of acryloyl esters

Acryloyl chloride (4-5 equiv.) was added to the mixture of a respective merocyanine dye (1 equiv.) and Et₃N (4-5 eq.) in dry DCM at 0°C under Ar. After 1 h of stirring at 0°C, the reaction was quenched by adding aqueous NH4Cl (0.1 M), and organic materials were extracted twice with DCM. The combined extracts were washed with NH4Cl (0.1 M), dried over Na2SO4, and concentrated *in vacuo.* The residue was purified by reversed phase column chromatography (C-18, H2O/MeCN).

### General procedure III

### Preparation of acryloyl esters

Acryloyl chloride (4-5 equiv.) was added to the mixture of a respective merocyanine dye (1 equiv.) and Et₃N (4-5 eq.) in dry DCM at 0°C under Ar. After 1 h of stirring at 0°C, the reaction was quenched by adding aqueous NH₄Cl (0.1 M), and water-soluble product was extracted twice with H₂O. The combined extracts were washed with DCM, concentrated *in vacuo* (20 mbar, 20°C), then purified by reversed phase column chromatography (C-18, H₂O/MeCN).

### Spectroscopic Materials and Methods

Absorption spectra were recorded on a Cary 50 UV-vis spectrometer from Varian. All measurements were performed in 1 cm UV-vis disposable cuvettes (BRAND semi-micro) and air-equilibrated solutions at 25 ± 0.1 °C. A total assay volume of 1.0 mL was used for each measurement. UV-vis scan spectra were recorded using following parameters: average time 0.05 s; data interval 1 nm; scan rate 1200 nm/min; with base line correction.

Solutions were prepared in 1.5 mL-vials (Eppendorf^{®} microtubes 3810X) using Vortex Mixers.

Stock solutions of assayed compounds (2-5 mM) were prepared in H₂O-DMSO (1:1), stored at -20°C, and diluted to 1.0 mM with buffer before use. The L-Cys stock solution (20.0 mM) was freshly prepared in buffer before the measurements. HEPES buffer (25 mM, pH 7.4) was used for all measurements.

All aqueous solutions were made up in deionized water with resistivity ≥ 18 MΩ cm⁻¹, obtained using a Millipore purification system (MQ-water).²

### Extinction coefficients

For a measurement, 1000 µL of buffer and 1-50 µL of probe (1.0 mM) were mixed, and then transferred to a cuvette. Absorbance spectra (250-800 nm) were measured against a blank of the buffer. At least six concentrations of each compound were used to calculate extinction coefficients from the slope of probe concentration vs absorbance plots, using MS Excel software (Microsoft).

In the same manner, ε^{Cys} was determined from the solutions of probe reacted with an excess of Cys (100 µM). The reactions were performed at 37 °C (incubation time 15 min). Blank reactions were run without addition of Cys.

### Evaluation of Stability

For a measurement, 1000 µL of buffer and 16 µL of probe (1.0 mM) were mixed, and then incubated at +4 °C and at +37 °C for 5 h. The resulting mixtures were transferred to a cuvette, and the absorbance (250-800 nm) was measured. Each measurement was done in triplicate.

### Evaluation of Solubility

In the experiments, 5-10 mg of dried material was suspended in 250-500 µL of H2O at RT. The resulting suspension was centrifuged for 10 min at RT (16000 rcf). UV-vis of supernatant were recorded in buffer (25 mM HEPES pH 7.4) at RT. Each measurement was done in triplicate. The pellet was dried *in vacuo* for 16 h, and then weighed.

As mentioned above, the final products were obtained in a two-step synthesis (condensation and acrylation; Schemes 1 to 3). Overall yields 21-59%, except for 6% in case of MF65. The products were characterized by HPLC-MS, ¹H and ¹³C NMR, and UV-Vis.

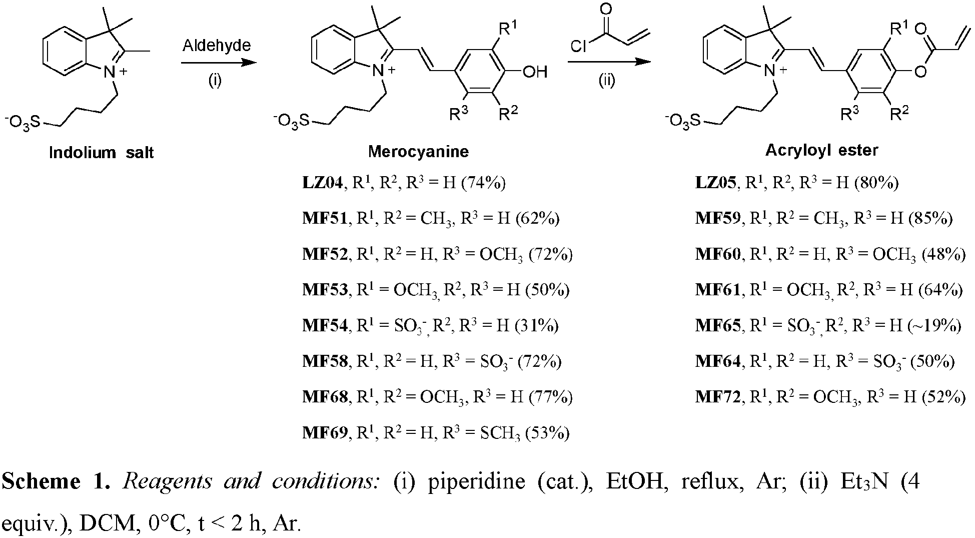

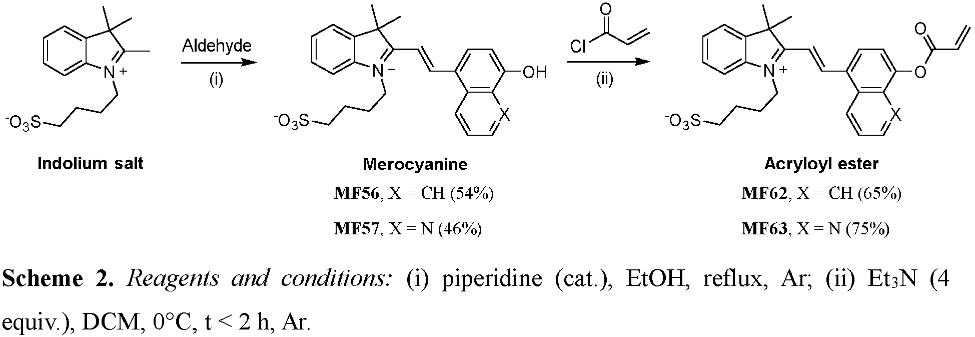

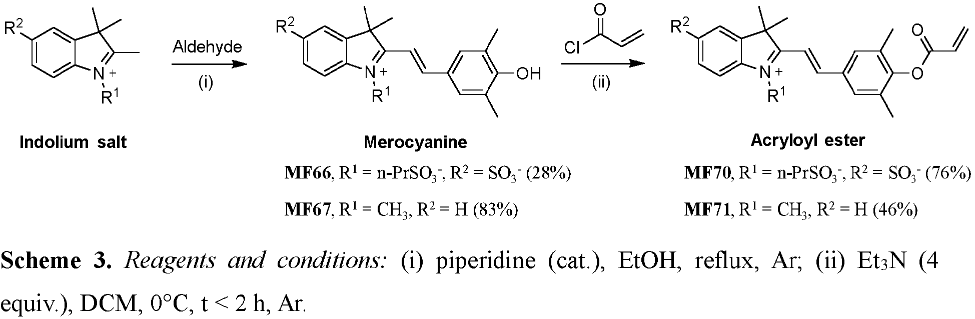

### UV-vis and Cys Response

UV-vis of dyes as obtained was evaluated. Selected substituents on the benzene ring helped to increase the value for the initial merocyanine dye. Most remarkable results were observed for the preferred intermediate compounds **MF56, MF57** and **MF66.**

**Table 1. Spectral properties of dyes according to the present invention.***

| Entry | λₘₐₓ (ε mM⁻¹cm⁻¹) nm |
|---|---|
| **LZ04** | 527 (36.6) |
| **(contr.)** | 527 (45.6) ** |
| **LZ06** | 520 (27-37) **I⁻ not quant.** |
| **(contr.)** | 520 (37-53) **I⁻ not quant.** |
| **MF51** | 550 (73.9) |
| **MF52** | 529 (70.1) |
| **MF53** | 548 (65.3) |
| **MF54** | 528 (44.9) |
| **MF56** | 582 (109.7) |
| **MF57** | 572 (106.3) |
| **MF58** | 514 (48.1, broad) |
| **MF66** | 556 (122.6) |
| | 556 (138.6)** |
| **MF67** | 546 (67.4) |
| | 546 (84.9)** |
| **MF68** | 575 (70.0) |

| | |
|---|---|
| *UV-vis spectra were recorded in aqueous buffer (25 mM HEPES pH 7.4). The concentration of the probes was 1-24 µM. At least six concentrations of each compound were used in the experiments. **The experiments were performed in the same buffer at pH 8.0. | |

Spectra recorded in the presence of Cys confirmed a colorimetric response through the cleavage of acryloyl ester. Probes as synthesized showed significant bathochromic shifts to the green and yellow range of the visible spectrum (Table 2). Moreover, their spectral profiles were advantageously fulfilling the wavelength requirement for the Cys sensing application in Cedex system (340, 378, 409, 480, 512, 520, 552, 583, 629, 652, 659 and 800 nm).

**Table 2. Spectral properties and Cys-response screening of compounds according to the present invention. ***

| Entry | λₘₐₓ (ε mM⁻¹cm⁻¹) nm | λ^{Cys} (ε^{Cys} mM⁻¹cm⁻¹) nm | SNR |
|---|---|---|---|
| **LZ05 (contr.)** | 527 (1.3), 389 (17.3) | 527 (31.9) | 12 |
| **LZ07 (contr.)** | 520 (0.6), 383 (16.9) | 520 (31.5) | 16 |
| **MF59** | 550 (0.7), 391 (16.7) | 550 (17.9) | 20 |
| | | 550 (52.9)** | 44** |
| **MF60** | 530 (2.1), 409 (14.3) | 529 (67.8) | 14 |
| **MF61** | 548 (0.4), 397 (20.4) | 549 (61.7) | 36 |
| **MF62** | 582 (1.5), 419 (8.5), 287 (7.7) | 582 (105.3) | 20 |
| **MF63** | 572 (2.9), 397 (14.0) | 572 (93.4) | 10 |
| **MF64** | 514 (0.7), 350 (8.0) | 514 (32.1) | 14 |
| **MF65** | 528 (1.2), 381 (17.7) | 528 (44.3) | 16 |
| **MF70** | 400 (21.9) | 556 (23.6) | 118 |
| | 400 (16.6)* | 556 (55.8)** | 140** |
| **MF71** | 546 (0.6), 387 (29.1) | 547 (33.7) | 48 |
| | 546 (0.8), 385 (25.7)* | 547 (69.6)** | 53** |
| **MF72** | 396 (14.2) | 575 (31.0) | 103 |

| | | | |
|---|---|---|---|
| *UV-vis spectra were recorded before and after addition of an excess of Cys (100 µM) in aqueous buffer (25 mM HEPES pH 7.4). The concentration of the probes was 1-24 µM. The reactions with Cys were performed at 37 °C (incubation time 15 min). SNR was estimated from the values obtained after reaction with Cys (ε^{Cys}) and of blank measurements (ε^{blank}). At least six concentrations of each compound were used in the experiments. **The experiments were performed in the same buffer at pH 8.0. | | | |

### Stability

The stability is another important parameter for the evaluation of colorimetric probes for biological assays. The probes for commercial applications have to be storable at 4 °C for several months. In addition, the stability has to be examined under assay conditions of Cedex Bio HT analyzer (37 °C).

The inventors qualitatively examined the stability of probes in buffer (10 mM HEPES pH 7.4) at 4 °C and 37 °C in order to simulate usual conditions of storage and assays. Solutions of each probe were monitored by UV-vis for 5 h. The results of all spectroscopic evaluations are summarized in Table 3.

**Table 3.* Stability of compounds of the present invention**

| Entry | Hydrolysis @4°C, % | Hydrolysis @37°C, % |
|---|---|---|
| **LZ05 (contr.)** | 8.4 ± 0.2 | 36.9 ± 0.4 |
| **LZ07 (contr.)** | 7.3 ± 0.1** | 30.1 ± 0.2** |
| **MF59** | 1.2 ± 0.0 | 4.7 ± 0.1 |
| **MF60** | 9.8 ± 0.3 | 41.8 ± 0.9 |
| **MF61** | 4.8 ± 0.1 | 29.1 ± 1.9 |
| **MF62** | 7.6 ± 0.5 | 40.4 ± 0.6 |
| **MF63** | 9.8 ± 1.5 | 55.3 ± 0.7 |
| **MF64** | 8.9 ± 0.2 | 45.2 ± 1.4 |
| **MF65** | 7.2 ± 0.2 | 28.9 ± 1.4 |
| **MF70** | 0.2 ± 0.0 | 0.8 ± 0.0 |
| **MF71** | 1.0 ± 0.1 | 2.3 ± 0.2 |
| **MF72** | 0.8 ± 0.1 | 2.8 ± 0.1 |

| | | |
|---|---|---|
| *The experiments were performed in aqueous buffer (25 mM HEPES pH 7.4) at 4 °C and 37 °C, with a concentration of 15.6 µM (incubation time 5 h). The amount of hydrolyzed probe was determined using ε values derived from merocyanine analogues **(Table 1).** Each measurement was done in triplicate. **The amount of hydrolyzed probe was determined using ε value derived from Cys response screening (Table 2). | | |

### Solubility

The aqueous solubility of probes is another important property for the performance of compounds in biological assays. The inventors determined the solubility in water of the probes according to the invention using UV-vis. First, oversaturated mixtures of each probe were prepared. The mixtures were centrifuged, and then the concentrations in probes of supernatant were examined by UV-vis. To further validate the results, the concentrations were calculated from the isolated pellet weight. As seen in Table 4, the results as determined with both approaches are in the same range, and followed the same trend. Preferred probe **MF70,** containing two sulpho-groups, is highly water-soluble in comparison to its analogues **LZ05** and **MF59** containing only one sulpho-group. Also, the solubility of **MF70** is superior to the compound **LZ07** known from literature.

**Table 4.* Solubility.**

| Entry | Solubility (by UV-vis), mM | Solubility (by weight), mM |
|---|---|---|
| **LZ05 (contr.)** | 11.0 ± 1.0 | 12 |
| **LZ07 (contr.)** | 15.4 ± 1.5 | 17 |
| **MF59** | 5.8 ± 0.2 | 2 |
| **MF70** | ≥41 | ≥47 |

| | | |
|---|---|---|
| *In the experiments 5-10 mg of dried material was suspended in 250-500 µL of H₂O at RT. The UV-vis of supernatants was recorded in buffer (25 mM HEPES pH 7.4) at RT. Each measurement was done in triplicate. Probes were used as obtained from synthesis. | | |

### Probe MF70 Performance in Cedex Bio HT

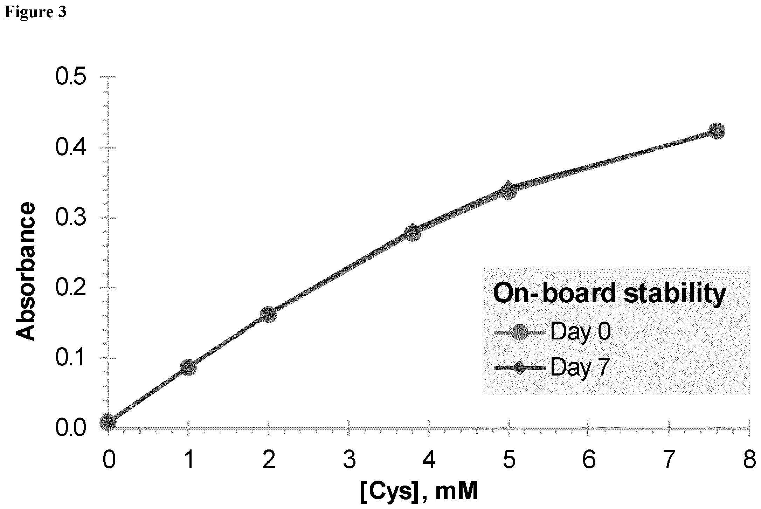

The rapid kinetic profile, stability and low background signal as obtained were encouraging to further use **MF70** for Cys-sensing in Cedex Bio HT. Reagent solution was treated with various Cys-concentrations (0.5-7.6 mM) in a feed medium, which is used for monoclonal antibody production. As shown in Figure 3, progressively enhanced absorbance was observed with the increasing amount of Cys. Under these conditions, a reliable response was obtained over the period of one week (Table 5), with corresponding detection limit of 3.6 µM Cys, and limit of blank 2.2 µM Cys.

**Table 1. Spike-recovery in a feed medium (DMT118F.01 with Cys).**

| Spiked Cys, mM | n (samples) | Found, mM | CV, % | Recovery, % |
|---|---|---|---|---|
| 0.5 | 21 | 0.64 | 1 | 96 |
| 1.5 | 21 | 1.64 | 1 | 99 |
| 3.0 | 21 | 3.03 | 1 | 96 |
| 4.5 | 21 | 4.38 | 1 | 94 |
| 6.0 | 21 | 5.83 | 1 | 94 |

In conclusion, the probes according to the present invention, and preferably probe MF70 meet the requirements for commercial assays. The merocyanine dye scaffold ensures bright chromogenic signal. Methyl groups in the ortho-position and sulfonic acid groups seem to secure stability against hydrolysis and aqueous solubility, respectively.

## Claims

1. A compound according to Formula I 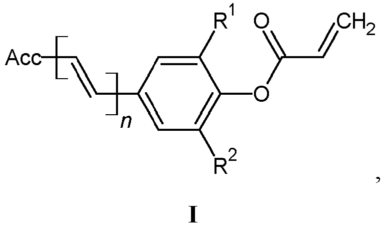 wherein
R¹ and R² are independently selected from R³, O-R³, S-R³, SO₃⁻, SO₃-R³, wherein R³ is selected from Ci-Cis alkyl, and a polyethylene glycol (PEG) residue,
Acc is selected from the group selected from Formula II 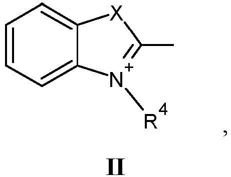
wherein X is selected from -N(CH₃)-, -S-, -Se-, -O-, and -C(CH₃)₂-, 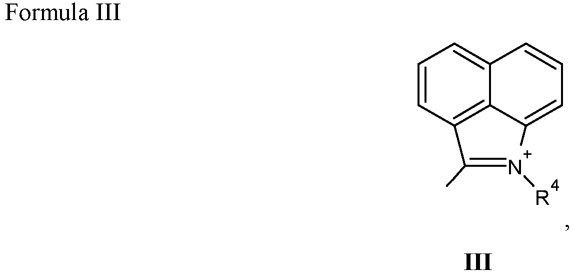 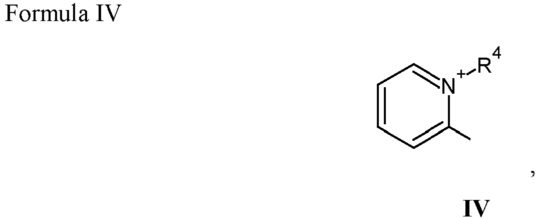 and 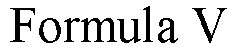 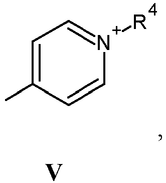
wherein optionally in each of Formula II to V an aromatic ring is substituted with 1, 2 or 3 SO₃⁻ groups,
R⁴ is selected from the group of C₁-C₁₈ alkyl, C₁-C₆ cycloalkyl, and (CH₂)ₘ-SO₃⁻, wherein m is an integer selected from 1 to 18, and
n is selected from 1, 2 and 3,
and suitable salts and solvates thereof.

2. The compound of Formula I according to claim 1, wherein R¹ and R² are independently selected from R³, O-R³, S-R³, SO₃⁻, SO₃-R³, wherein R³ is selected from C₁-C₆ alkyl, and a polyethylene glycol (PEG) residue,
Acc is Formula II 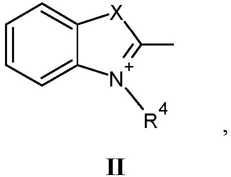
wherein X is selected from -N(CH₃)-, -S-, -Se-, -O-, and -C(CH₃)₂-, optionally the aromatic ring is substituted with 1, 2 or 3 SO₃⁻ groups, R⁴ is selected from C₁-C₆ alkyl, C₁-C₆ cycloalkyl, and (CH₂)ₘ-SO₃⁻, wherein m is an integer from 1 to 6, and n is 1, and suitable salts and solvates thereof.

3. The compound of Formula I according to claim 1, wherein R¹ and R² are independently selected from R³, O-R³, S-R³, SO₃⁻, SO₃-R³, wherein R³ is selected from C₁-C₃ alkyl, and a polyethylene glycol (PEG) residue,
Acc is Formula II 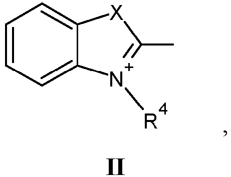
wherein X is -C(CH₃)₂-, optionally the aromatic ring is substituted with 1, 2 or 3 SO₃⁻ groups, R⁴ is (CH₂)ₘ-SO₃⁻, wherein m is an integer from 1 to 6, and n is 1, and suitable salts and solvates thereof.

4. The compound of Formula I according to the following formulae VI to IX 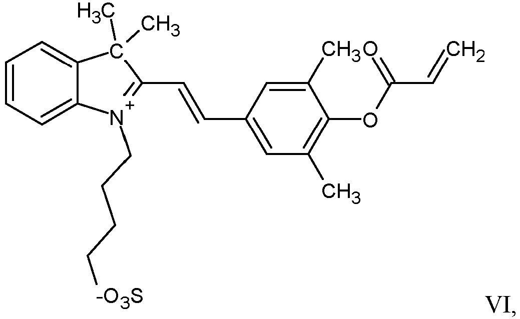 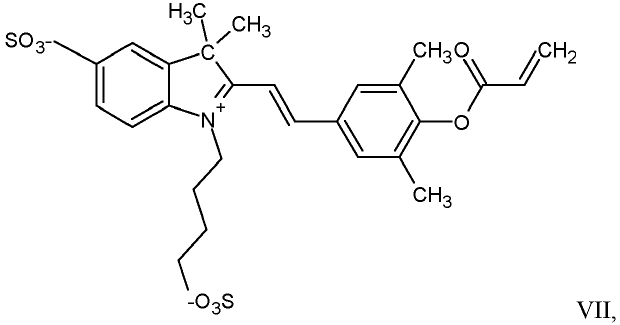 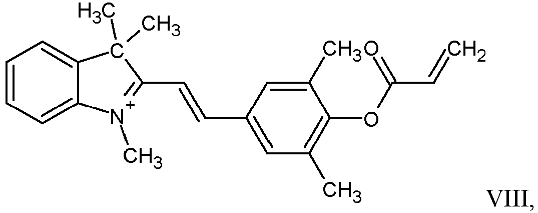 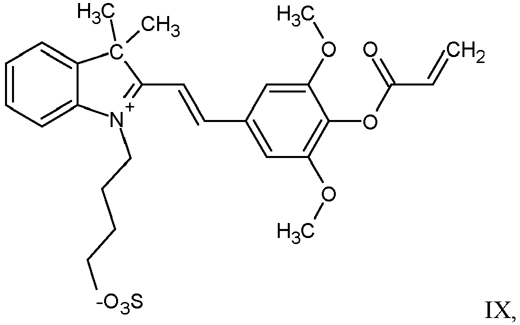 and suitable salts and solvates thereof.

5. A method for preparing a compound according to Formula I according to claim 1, comprising the steps of:
a) suitably reacting a compound of Formula VI 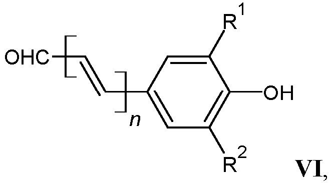 wherein R¹ and R² are as defined in claim 1, and n is 1 or 2, with a compound of Formula II 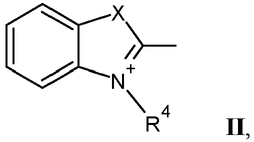 or with a compound of formula III 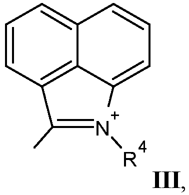 or with a compound of Formula IV 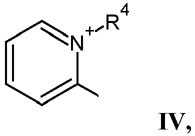 or with a compound of Formula V 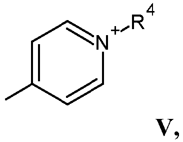 wherein in each of Formulae II to V optionally an aromatic ring is substituted with 1, 2 or 3 SO₃⁻ groups, R⁴ is selected from C₁-C₁₈ alkyl, C₁-C₆ cycloalkyl, and (CH₂)ₘ-SO₃⁻, and wherein m is an integer from 1 to 18, to obtain a compound of Formula VIII 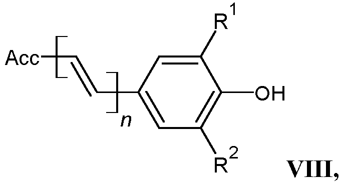 wherein R¹, R² and Acc are as defined in claim 1, and n is 1 or 2, and
b) suitably reacting the compound of formula VIII with acryloyl chloride.

6. A method for detecting cysteine in a test sample, comprising the following steps of
a) Measuring of UV/Vis absorbance of a solution of a compound as defined in any one of claims 1 to 4 in a suitable solvent before and after being contacted with a prospectively cysteine-containing test sample, and
b) Determining the difference in absorbance by comparison of the UV/Vis spectra as measured in step a), and
c) Detecting cysteine in said test sample based on said difference in absorbance as determined in step b).

7. The method according to claim 5, wherein the UV/Vis absorbance is measured at discrete wavelengths in the range of from 200 nm to 1000 nm.

8. The method according to claim 7, wherein the wavelengths are selected from the group consisting of 340, 378, 409, 480, 512, 520, 552, 583, 629, 659 and 800 nm.

9. The method according to any one of claims 6 to 8, wherein the solvent is an aqueous solvent.

10. The method according to any one of claims 6 to 9, wherein said determining the difference in absorbance is by a visual inspection of a color change.

11. The method according to any one of claims 6 to 9, wherein said determining the difference in absorbance is by Cedex

12. A kit for detecting cysteine in a test sample, comprising a vial or container comprising a predetermined quantity of a compound according to any one of claims 1 to 4, together with a manual for using said kit.

13. Use of a compound according to any one of claims 1 to 4, or a kit according to claim 12 for detecting cysteine in a test sample, preferably an aqueous test sample.

## Patentansprüche

1. Verbindung gemäß Formel I 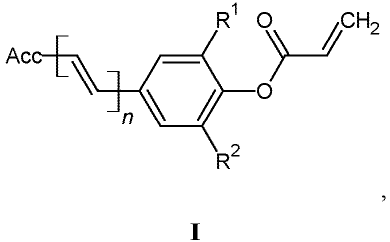 wobei
R¹ und R² unabhängig voneinander aus R³, O-R³, S-R³, SO₃⁻, SO₃-R³ ausgewählt sind, wobei R³ aus C₁-C₁₈-Alkyl und einem Polyethylenglykol-(PEG-)Rest ausgewählt ist,
Acc aus der Gruppe ausgewählt ist, die aus Formel II 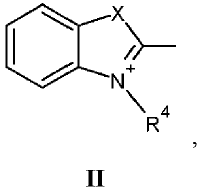
wobei X aus -N(CH₃)-, -S-, -Se-, -O- und -C(CH₃)₂- ausgewählt ist; 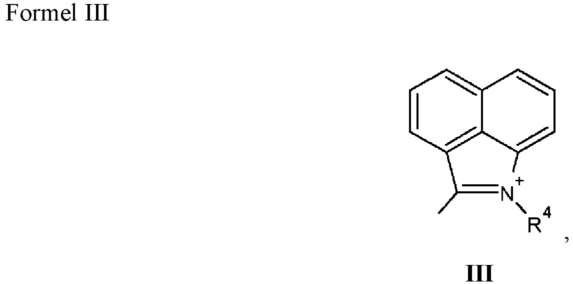 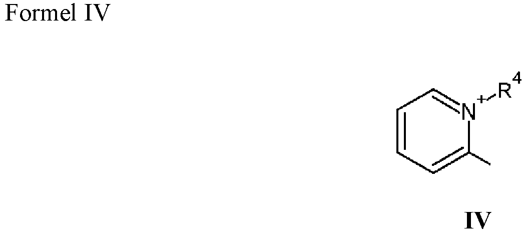
und 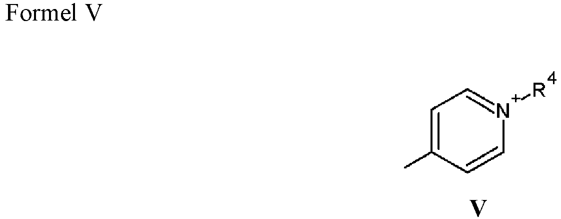
ausgewählt ist, wobei gegebenenfalls in jeder der Formeln II bis V ein aromatischer Ring mit 1, 2 oder 3 SO₃⁻-Gruppen substituiert ist,
R⁴ aus der Gruppe von C₁-C₁₈-Alkyl, C₁-C₆-Cycloalkyl und (CH₂)ₘ-SO₃⁻ ausgewählt ist, wobei m eine ganze Zahl ist, die aus 1 bis 18 ausgewählt ist und
n aus 1, 2 und 3 ausgewählt ist,
und geeignete Salze und Solvate davon.

2. Verbindung der Formel I nach Anspruch 1, wobei R¹ und R² unabhängig voneinander aus R³, O-R³, S-R³, SO₃⁻, SO₃-R³ ausgewählt sind, wobei R³ aus C₁-C₆-Alkyl und einem Polyethylenglykol-(PEG-)Rest ausgewählt ist,
Acc Formel II ist 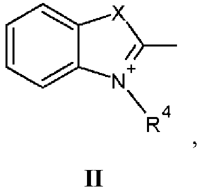
wobei X aus -N(CH₃)-, -S-, -Se-, -O- und -C(CH₃)₂- ausgewählt ist, gegebenenfalls der aromatische Ring mit 1, 2 oder 3 SO₃⁻-Gruppen substituiert ist, R⁴ aus C₁-C₆-Alkyl, C₁-C₆-Cycloalkyl und (CH₂)ₘ-SO₃⁻ ausgewählt ist, wobei m eine ganze Zahl von 1 bis 6 ist und n 1 ist, und geeignete Salze und Solvate davon.

3. Verbindung der Formel I nach Anspruch 1, wobei R¹ und R² unabhängig voneinander aus R³, O-R³, S-R³, SO₃⁻, SO₃-R³ ausgewählt sind, wobei R³ aus C₁-C₃-Alkyl und einem Polyethylenglykol-(PEG)-Rest ausgewählt ist,
Acc Formel II ist 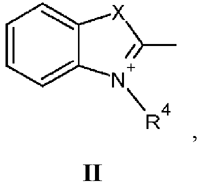
wobei X -C(CH₃)₂- ist, gegebenenfalls der aromatische Ring mit 1, 2 oder 3 SO₃⁻-Gruppen substituiert ist, R⁴ (CH₂)ₘ-SO₃⁻ ist, wobei m eine ganze Zahl von 1 bis 6 ist und n 1 ist, und geeignete Salze und Solvate davon.

4. Verbindung der Formel I gemäß den folgenden Formeln VI bis IX 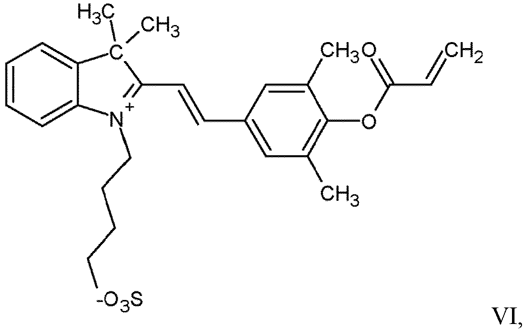 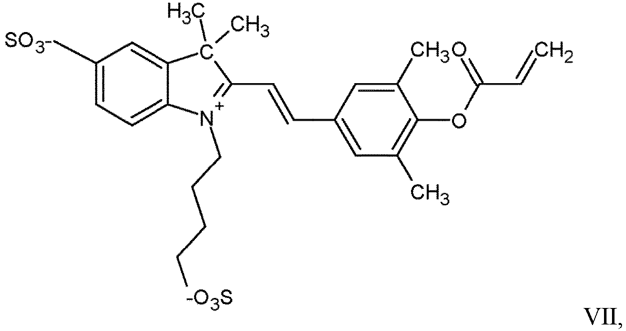 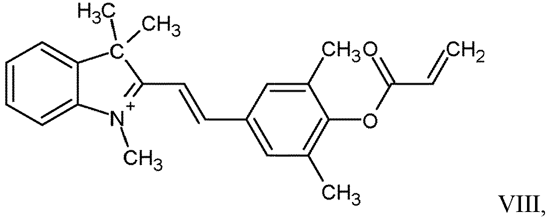 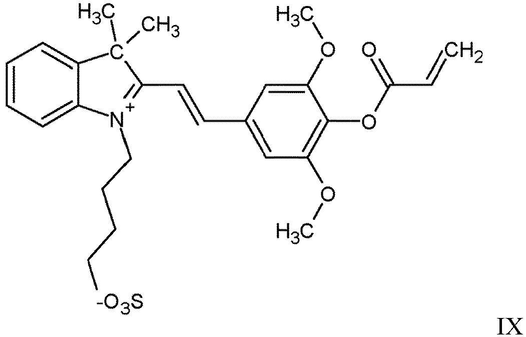 und geeignete Salze und Solvate davon.

5. Verfahren zur Herstellung einer Verbindung gemäß Formel I nach Anspruch 1, umfassend die folgenden Schritte:
a) geeignetes Reagieren einer Verbindung der Formel VI 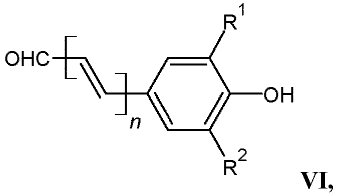 wobei R¹ und R² wie in Anspruch 1 definiert sind und n 1 oder 2 ist, mit einer Verbindung der 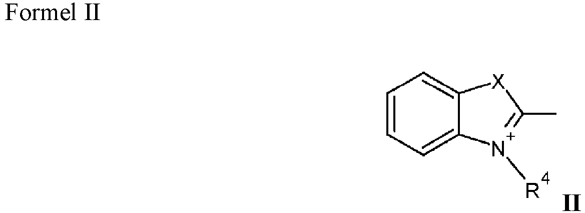 oder mit einer Verbindung der Formel III 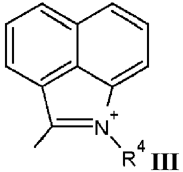 oder mit einer Verbindung der Formel IV 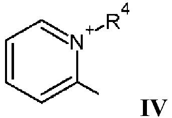 oder mit einer Verbindung der Formel V 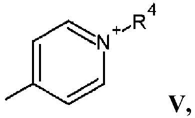 wobei in jeder der Formeln II bis V gegebenenfalls ein aromatischer Ring mit 1, 2 oder 3 SO₃⁻ -Gruppen substituiert ist, R⁴ aus C₁-C₁₈-Alkyl, C₁-C₆-Cycloalkyl und (CH₂)ₘ-SO₃⁻ ausgewählt ist und wobei m eine ganze Zahl von 1 bis 18 ist, unter Erhalt einer Verbindung der 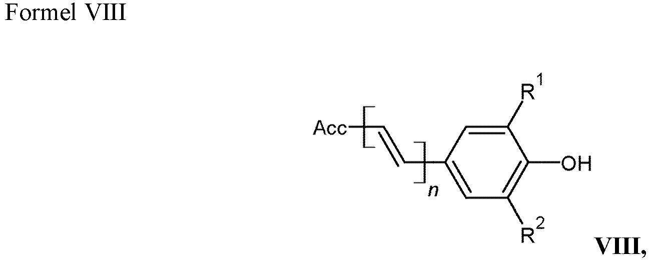 wobei R¹, R² und Acc wie in Anspruch 1 definiert sind und n 1 oder 2 ist, und
b) geeignetes Reagieren der Verbindung der Formel VIII mit Acryloylchlorid.

6. Verfahren zum Nachweisen von Cystein in einer Testprobe, umfassend die folgenden Schritte:
a) Messen der UV/Vis-Extinktion einer Lösung von einer Verbindung wie in einem der Ansprüche 1 bis 4 definiert in einem geeigneten Lösungsmittel, bevor und nachdem sie mit einer potenziell Cystein enthaltenden Testprobe kontaktiert worden ist, und
b) Bestimmen der Differenz in der Extinktion durch Vergleich der in Schritt a) gemessenen UV/Vis-Spektren und
c) Nachweisen von Cystein in der Testprobe basierend auf der in Schritt b) bestimmten Differenz in der Extinktion.

7. Verfahren nach Anspruch 5, wobei die UV/Vis-Extinktion bei diskreten Wellenlängen im Bereich von 200 nm bis 1000 nm gemessen wird.

8. Verfahren nach Anspruch 7, wobei die Wellenlängen aus der Gruppe ausgewählt sind, die aus 340, 378, 409, 480, 512, 520, 552, 583, 629, 659 und 800 nm besteht.

9. Verfahren nach einem der Ansprüche 6 bis 8, wobei das Lösungsmittel ein wässriges Lösungsmittel ist.

10. Verfahren nach einem der Ansprüche 6 bis 9, wobei das Bestimmen der Differenz in der Extinktion durch eine visuelle Prüfung einer Farbänderung erfolgt.

11. Verfahren nach einem der Ansprüche 6 bis 9, wobei das Bestimmen der Differenz in der Extinktion durch Cedex erfolgt.

12. Kit zum Nachweisen von Cystein in einer Testprobe, umfassend ein Fläschchen oder einen Behälter, das/der eine vorbestimmte Menge einer Verbindung nach einem der Ansprüche 1 bis 4 umfasst, zusammen mit einer Gebrauchsanleitung des Kits.

13. Verwendung einer Verbindung nach einem der Ansprüche 1 bis 4 oder eines Kits nach Anspruch 12 zum Nachweisen von Cystein in einer Testprobe, vorzugsweise einer wässrigen Testprobe.

## Revendications

1. Composé selon la formule I 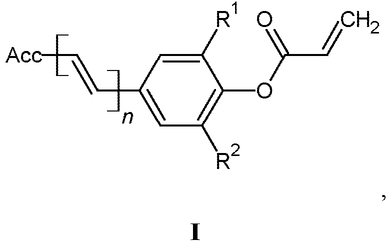 dans lequel
R¹ et R² sont indépendamment choisis parmi R³, O-R³, S-R³, SO₃⁻, SO₃-R³, dans lequel R³ est choisi parmi un alkyle en C₁-C₁₈ et un résidu polyéthylène glycol (PEG),
Acc est choisi dans le groupe choisi parmi la formule II 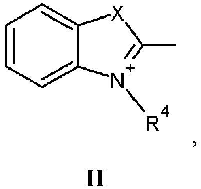
dans lequel X est choisi parmi -N(CH₃)-, -S-, -Se-, -O- et -C(CH₃)₂-,
la formule III 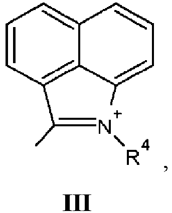 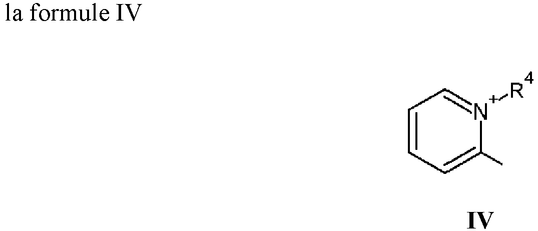 et 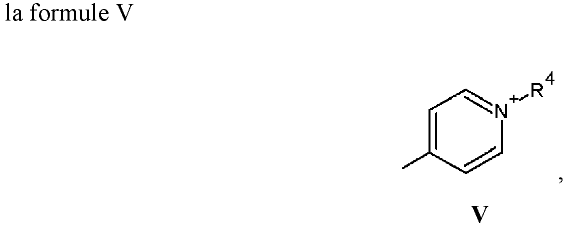
dans lequel éventuellement dans chacune des formules II à V un cycle aromatique est substitué par 1, 2 ou 3 groupes SO₃⁻,
R⁴ est choisi dans le groupe constitué par un alkyle en C₁-C₁₈, un cycloalkyle en C₁-C₆ et (CH₂)ₘ-SO₃⁻, dans lequel m représente un nombre entier choisi parmi 1 à 18, et
n est choisi parmi 1, 2 et 3,
et des sels et solvates appropriés de celui-ci.

2. Composé de formule I selon la revendication 1, dans lequel R¹ et R² sont indépendamment choisis parmi R³, O-R³, S-R³, SO₃⁻, SO₃-R³, dans lequel R³ est choisi parmi un alkyle en C₁-C₆ et un résidu polyéthylène glycol (PEG),
Acc représente la formule II 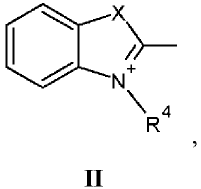
dans lequel X est choisi parmi -N(CH₃)-, -S-, -Se-, -O- et -C(CH₃)₂-, éventuellement le cycle aromatique est substitué par 1, 2 ou 3 groupes SO₃⁻, R⁴ est choisi parmi un alkyle en C₁-C₆, un cycloalkyle en C₁-C₆ et (CH₂)ₘ-SO₃⁻, dans lequel m représente un nombre entier de 1 à 6, et n représente 1, et des sels et solvates appropriés de celui-ci.

3. Composé de formule I selon la revendication 1, dans lequel R¹ et R² sont indépendamment choisis parmi R³, O-R³, S-R³, SO₃⁻, SO₃-R³, dans lequel R³ est choisi parmi un alkyle en C₁-C₃ et un résidu polyéthylène glycol (PEG),
Acc représente la formule II 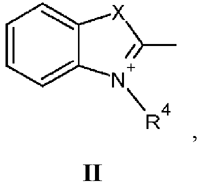
dans lequel X représente -C(CH₃)₂-, éventuellement le cycle aromatique est substitué par 1, 2 ou 3 groupes SO₃⁻, R⁴ représente (CH₂)ₘ-SO₃⁻, dans lequel m représente un nombre entier de 1 à 6, et n représente 1, et des sels et solvates appropriés de celui-ci.

4. Composé de formule I selon les formules VI à IX suivantes 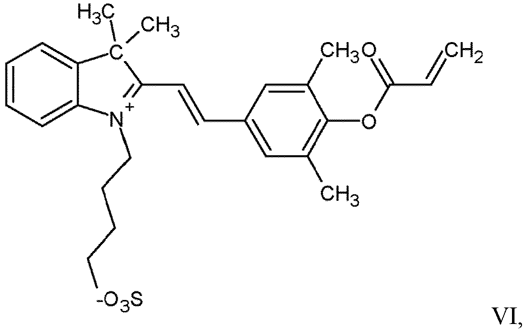 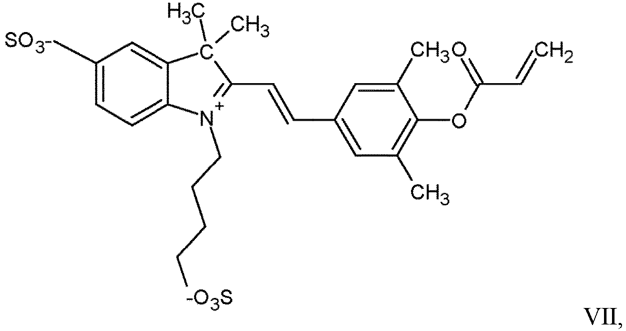 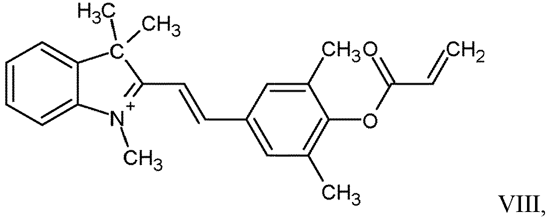 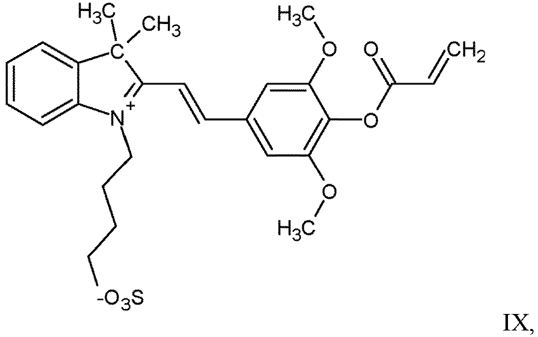 et des sels et solvates appropriés de celui-ci.

5. Procédé de préparation d'un composé selon la formule I selon la revendication 1, comprenant les étapes de :
a) réaction de manière appropriée d'un composé de formule VI 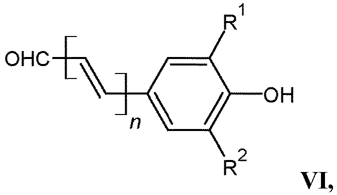 dans lequel R¹ et R² sont tels que définis dans la revendication 1 et n représente 1 ou 2, avec un composé de formule II 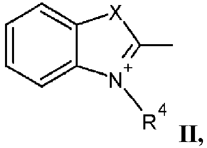 ou avec un composé de formule III 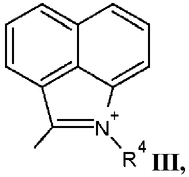 ou avec un composé de formule IV 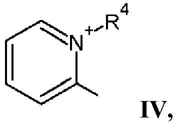 ou avec un composé de formule V 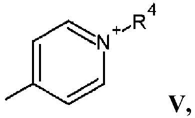 dans lequel dans chacune des formules II à V éventuellement un cycle aromatique est substitué par 1, 2 ou 3 groupes SO₃⁻, R⁴ est choisi parmi un alkyle en C₁-C₁₈, un cycloalkyle en C₁-C₆ et (CH₂)ₘ-SO₃⁻, et dans lequel m représente un nombre entier de 1 à 18, pour obtenir un composé de formule VIII 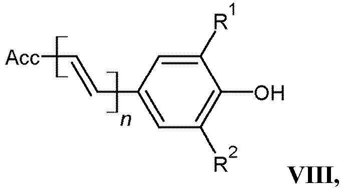 dans lequel R¹, R² et Acc sont tels que définis dans la revendication 1 et n représente 1 ou 2, et
b) réaction de manière appropriée du composé de formule VIII avec un chlorure d'acryloyle.

6. Procédé de détection de cystéine dans un échantillon de test, comprenant les étapes suivantes de :
a) mesure de l'absorbance UV/Vis d'une solution d'un composé tel que défini dans l'une quelconque des revendications 1 à 4 dans un solvant approprié avant et après avoir été mis en contact avec un échantillon de test contenant une cystéine de manière prospective, et
b) détermination de la différence d'absorbance par comparaison des spectres UV/Vis tels que mesurés à l'étape a), et
c) détection de la cystéine dans ledit échantillon de test sur la base de ladite différence d'absorbance telle que déterminée à l'étape b).

7. Procédé selon la revendication 5, dans lequel l'absorbance UV/Vis est mesurée à des longueurs d'onde distinctes dans la plage allant de 200 nm à 1000 nm.

8. Procédé selon la revendication 7, dans lequel les longueurs d'onde sont choisies dans le groupe constitué par 340, 378, 409, 480, 512, 520, 552, 583, 629, 659 et 800 nm.

9. Procédé selon l'une quelconque des revendications 6 à 8, dans lequel le solvant est un solvant aqueux.

10. Procédé selon l'une quelconque des revendications 6 à 9, dans lequel ladite détermination de la différence d'absorbance est par inspection visuelle d'un changement de couleur.

11. Procédé selon l'une quelconque des revendications 6 à 9, dans lequel ladite détermination de la différence d'absorbance est par Cedex.

12. Kit de détection de cystéine dans un échantillon de test, comprenant un flacon ou un récipient comprenant une quantité prédéterminée d'un composé selon l'une quelconque des revendications 1 à 4, conjointement avec un manuel d'utilisation dudit kit.

13. Utilisation d'un composé selon l'une quelconque des revendications 1 à 4, ou d'un kit selon la revendication 12 pour la détection de cystéine dans un échantillon de test, de préférence un échantillon de test aqueux.
